# EUROPEAN PATENT SPECIFICATION

(11) **EP 0 439 202 B1**
(45) Date of publication and mention of the grant of the patent: **29.09.1993**
(21) Application number: 91104476.6
(22) Date of filing: 02.02.1990
(51) Int. Cl.: B29C 49/14, B29C 49/16, B29C 49/64, A61M 25/00, B29L 23/00, A61M 29/00

(54) **Apparatus and method for manufacturing balloons for medical devices**
Vorrichtung und Verfahren zur Herstellung von Ballonen für medizinische Geräte
Appareil et procédé pour la fabrication de ballons pour dispositifs médicaux

(30) Priority: 24.07.1989 US 384418
(43) Date of publication of application: 31.07.1991
(62) Divisional of application: 90102115.4
(73) Proprietor: Cordis Corporation, Miami Lakes Florida 33014 (US)
(72) Inventor: Jackowski, Stefan, Hollywood, Florida 33024 (US); Pinchuk, Leonard, Miami, Florida 33186 (US)
(74) Representative: KUHNEN, WACKER & PARTNER

(56) References cited:
- EP-A- 0 052 490
- EP-A- 0 274 411
- US-A- 4 130 617
- US-A- 4 490 421

## Description

### Background and Description of the Invention

The present invention generally relates to apparatus and methods for manufacturing balloons for medical devices. More particularly, the invention relates to the manufacture of medical or surgical balloons and to catheters incorporating them, such as those designed for angioplasty, valvuloplasty and urological uses and the like. The balloons exhibit the ability to be tailored to have expansion properties which are desired for a particular end use. The expansion property of particular interest is the amount or percentage of expansion or stretching beyond a non-distended inflated size at which the balloons are inflated to remove folding wrinkles but are not stretched. Balloons which are especially suitable in this regard are made of nylon or polyamide tubing that has been formed into the desired balloon configuration.

Catheter balloons and medical devices incorporating them are well-known for use in surgical contexts such as angioplasty procedures and other medical procedures during which narrowings or obstructions in blood vessels and other body passageways are altered in order to increase blood flow through the obstructed area of the blood vessel. For example, in a typical balloon angioplasty procedure, a partially occluded blood vessel lumen is enlarged through the use of a balloon catheter that is passed percutaneously by way of the arterial system to the site of the vascular obstruction. The balloon is then inflated to dilate the vessel lumen at the site of the obstruction.

Essentially, a balloon catheter is a thin, flexible length of tubing having a small inflatable balloon at a desired location along its length, such as at or near its tip. Balloon catheters are designed to be inserted into a body passageway such as the lumen of a blood vessel, a heart passageway, a urological passageway and the like, typically with fluoroscopic guidance.

In the past, medical device balloon materials have included balloons having a wall thickness at which the material exhibits strength and flexibility that allow inflation to a working diameter or designated initial dilation diameter which, once achieved, is not surpassable to any significant degree without balloon breakage or substantially increasing the risk of balloon breakage. Balloons of these materials can be characterized as being substantially non-distensible balloons that are not stretchable, expandable or compliant to a substantial extent beyond this working diameter. Such substantially non-distensible balloons can be characterized as being somewhat in the nature of paper bags which, once inflated to generally remove folding wrinkles, do not further inflate to any significant degree. Polymeric materials of this substantially non-distensible type that are used or proposed for use as medical balloons include polyethylene terephthalates.

Other types of materials, such as polyvinyl chlorides and cross-linked polyethylenes can be characterized as being distensible in that they grow in volume or stretch with increasing pressure until they break. These materials are generally elastic and/or stretchable. When such extensible materials are used as medical balloons, the working diameter or designated dilation diameter of the balloon can be exceeded, based upon the stretchability of the material.

Substantially non-distensible balloons have at times been considered to be advantageous because they will not inflate significantly beyond their respective designated dilation diameters, thereby minimizing possible over-inflation errors. The theory is that one need only select a balloon such as an angiographic balloon that has an opened and inflated diameter which substantially corresponds to the dilation size desired at the obstruction site. However, physiological vessels such as arteries are generally tapered and do not always coincide with readily available catheter balloon dimensions, and at times it may be preferable to be able to increase the diameter of the balloon beyond what had been initially anticipated. With a substantially non-distensible balloon, such further extension is severely limited. On the other hand, certain non-distensible materials out of which medical balloons are made generally possess relatively high tensile strength values, which is typically a desirable attribute, especially for dilating tough lesions.

More readily distensible materials such as polyvinyl chloride typically exhibit a lower tensile strength and a larger elongation than a substantially non-distensible material such as polyethylene terephthalate. This relatively low tensile strength increases the risk of possible balloon failure. Due to their larger ultimate elongation, most readily distensible materials can provide a wide range of effective inflation or dilation diameters for each particular balloon size because the inflated working profile of the balloon, once achieved, can be further expanded in order to effect additional dilation. But this very property of having an expanded dilation range is not without its dangers because of the increased risk of overinflation that can damage the blood vessel being treated, and the overinflation risk may have to be compensated for by using balloon wall thicknesses greater than might otherwise be desired.

Although a material such as polyethylene terephthalate is advantageous from the point of view of its especially high tensile strength and its tightly controllable inflation characteristics, it has undesirable properties in addition to its general non-distensibility. In some situations, probably based on an excessive crystallinity of the polyethylene terephthalate, the balloon itself or the thicker leg sections thereof will not readily fold over and down in order to provide the type of low profile that is desirable for insertion through a guiding catheter and/or through the cardiovascular system and especially through the narrowed artery which is to be dilated. The resistance to folding, or "winging," is an especially difficult problem when it comes to larger balloons, such as those intended for valvuloplasty applications.

Also, it has been observed that thin-walled materials such as polyethylene terephthalate have a tendency to form pin holes or exhibit other signs of weakening, especially when flexed. Such a tendency can require extreme care in handling so as to avoid inadvertent damage that could substantially weaken a polyethylene terephthalate medical balloon. Although it is known that a lower profile and more flexible balloon and balloon legs can be made by thinning the wall, thus thinned polyethylene terephthalate balloons become extremely fragile and may not survive insertion through the cardiovascular system with desired integrity and without pin-holing and/or rupture.

Materials such as polyethylene terephthalate do not readily accept coating with drugs or lubricants, which can be desirable in many applications. Polyethylene terephthalate materials are also difficult to fuse, whether by adherence by heating or with known biocompatible adhesives.

The US-A-4 130 617 discloses a method for making a cuff or balloon for use with an endotracheal tube by means of a cold parison blow molding process. In carrying out the method, a thermoplastic or other elastomeric tube of predetermined dimensions and elasticity is prestretched a predetermined amount in the longitudinal direction and, then the tube is positioned within a hollow mold having the mold internal configuration in the general shape of the desired end balloon form.

Furthermore, US-A-4 490 421 discloses high molecular weight, biaxially oriented, flexible polymeric balloons for catheter applications and a process for manufacturing such balloons. The process comprises, at a temperature within the range extending from the second order transition temperature to the first order transition temperature, drawing a polymeric tubing having a finite length and an internal diameter which is about one half the outer diameter to a defined length, and thereafter expanding the drawn tubing.

In addition to this, EP-A-0 274 411 on which the preambles of independent claims 1 and 13 are based, discloses a method for forming a biaxially oriented flexible polymeric balloon comprising a tubular parison of orientable semicrystalline polymer, drawing said parison axially and thereafter expanding the drawn tubing radially by means of a pressurized fluid inside of the tubing.

It is a general object of the present invention to provide an improved apparatus and methods for fabricating a medical balloon.

An advantage of the present invention is to provide an improved apparatus and methods for forming medical balloons that exhibits a controlled distensibility at high pressure.

An other advantage of the present invention is to provide an improved apparatus and method for forming medical balloons which provide a range of dilatation capabilities while minimizing risks that could be associated with such flexibility.

Another advantage of the present invention is to provide an improved angioplasty catheter balloon molding method and apparatus that are especially suitable for forming tubing of tailorable materials such as nylon or polyamide into catheter balloons.

Another advantage of the present invention is to provide an improved means and method for radially expanding relatively amorphorous tubing into mechanically bidirectionally stretched medical balloons.

Another advantage of the present invention is to provide an improved apparatus and method for forming a balloon catheter that is readily coated with materials such as lubricating agents and the like which are advantageous for administration in association with balloon catheters.

Another advantage of this invention is to provide an improved method for forming a balloon that exhibits a combination of good strength and desirable flexibility.

Another advantage of this invention is to provide an improved apparatus and method for forming a medical balloon that exhibits an ability to be readily fused to other components.

By the present invention, these undesirable and/or disadvantageous properties of substantially non-distensible medical balloons such as those made from polyethylene terephthalate materials are significantly eliminated. At the same time, many of the advantages of these types of materials are provided or approximated. Furthermore, the present invention realizes many of the advantages of the more elastomeric materials such as polyvinyl chloride, but without the disadvantages of relatively low tensile strength, and the possibility of excessive expandability that can lead to overinflation of a blood vessel or the like.

In summary, the present invention achieves these objectives and provides advantageous properties along these lines by forming and providing a catheter and medical catheter balloon constructed of a material that is of limited and generally controlled distensibility whereby expansion beyond the working or fully expanded but non-distended dilation profile of the balloon is possible, but only to a desired extent which can, within limits, be tailored to the particular needs of the balloon device. The invention includes utilizing a tailorable material such as a nylon material or a polyamide material that is formed within the molding apparatus into a desired balloon by appropriate axial elongation, radial expansion as well as heat treatment procedures.

In particular, the present invention provides a molding apparatus for fabricating medical device balloons, the apparatus including a blow molding chamber having an infeed opening and an outfeed opening, means for feeding a parison through the infeed opening and outfeed opening of said blow molding chamber, means for axially elongating said parison in a first direction while a first portion of said parison is within said blow molding chamber means for supplying pressurized fluid into said parison and for effecting radial enlargement of said first portion of the parison within said blow molding chamber upon passage of pressurized fluid from the supplying means into said first portion of the parison, a free-blow molding chamber having an infeed opening and an outfeed opening upstream of said blow molding chamber,
said means for feeding the parison also feeds same through the infeed opening and the outfeed opening of said free-blow molding chamber,
said means for axially elongating said parison in a first direction also stretches said parison while a second portion thereof is within said free-blow molding chamber,
said means for supplying pressurized fluid supplies same at a location upstream of said infeed opening of the free-blow molding chamber,
said free-blow molding chamber has means for permitting stretching of a defined portion of said parison in a second direction by radially enlarging said second portion of the parison therewithin upon passage thereinto of pressurized fluid from said supplying means, and
means for thermally isolating said free-blow molding chamber from other components of the apparatus including said blow molding chamber.

Furthermore, the present invention provides a process for fabricating medical device balloons, the process including the steps of:
feeding an elongated parison through a molding apparatus including a blow molding chamber in which mechanically stretching of said parison in two directions can be achieved;
axially stretching to longitudinally elongate the parison within said blow molding chamber to provide an axially elongated parison length;
radially expanding said axially elongated parison length by infusing pressurized fluid thereinto to provide a radially expanded parison length; characterized in that:
said feeding step includes feeding the parison through a free-blow molding chamber upstream of the blow molding chamber;
axially stretching said parison within said free-blow molding chamber to longitudinally elongate said parison within said free-blow molding chamber;
blow molding said axially stretched parison length by infusing pressurized fluid thereinto while same is within the free-blow molding chamber to provide, together with said feeding, axially stretching and radially expanding steps, a molded parison in the form of a medical device balloon.

The above mentioned and other objects, features and advantages of this invention will be clearly understood through a consideration of the following detailed description.

### Brief Description of the Drawings

In the course of this description, reference will be made to the attached drawings, wherein:
Figure 1 is an elevational illustration, partially in cross-section, of a balloon catheter having a structure typical of that suitable for angioplasty;
Figures 2a, 2b, 2c, 2d and 2e are elevational illustrations of tubing as it is progressively processed to transform same into a medical balloon according to this invention;
Figure 3 is a cross-sectional view illustrating a preferred apparatus for carrying out the processing steps illustrated in Figures 2a, 2b, 2c, 2d and 2e;
Figure 4 is a plot of balloon size versus balloon inflation pressure for balloons made of a variety of materials, including nylon balloons tailored to provide different radial expansion properties;
Figures 5 and 6 are plots illustrating the function of heat setting on tailorability; and
Figure 7 is a plot illustrating the function of hoop expansion ratio on tailorability.

### Description of the Particular Embodiments

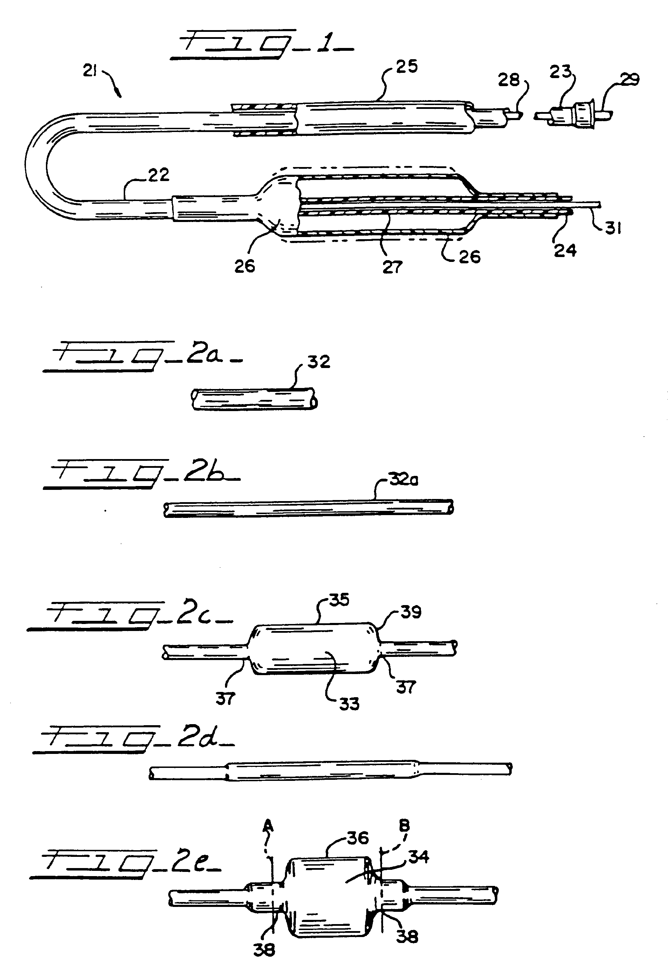

An illustrative catheter is generally designated in Figure 1 by reference numeral 21. Catheter 21 includes a catheter tube 22 having a proximal end 23 and a distal end 24. A guiding catheter 25 is also illustrated. A medical balloon 26 is shown secured to the distal portion of the catheter tube 22 in a location overlying one or more apertures 27 through the catheter tube. Extending through the lumen of the catheter tube 22 is an inner elongated body 28, such having a proximal end 29 and a distal tip end 31. The inner body 28 may be solid or have an internal lumen, depending upon the function that the inner body is to perform, whether it be simply a guiding function or whether it is intended to also provide the capability to insert materials into the bloodstream or measure parameters of the bloodstream, or the like.

Except for the balloon 26, all of these various components perform functions that are generally appreciated in the art. Typically with the aid of the guiding catheter 25, the inner body 28 and the catheter tube 22 are inserted into the cardiovascular system until the balloon is located at an occlusion site. At this stage, the balloon 26 is typically folded and collapsed, and it has an external diameter less than the inflated diameter illustrated in Figure 1, to the extent that the balloon 26 is generally wrapped around the catheter tube 22. Once the balloon 26 is maneuvered to the location of the occlusion, a pressurized fluid is inserted at the proximal end 23 of the catheter tube 22 for passage through aperture 27 and for inflation of the balloon 26. This unfolds the balloon until it presents a relatively smooth outer surface or working profile for imparting forces that are radially outwardly directed at the desired site within the body in order to achieve the desired result of lesion dilation, occlusion reduction or similar treatment. In accordance with an important aspect of the invention, the passage of greater pressure against the inside surface of the balloon 26 permits the outer surface of the balloon 26 to transmit additional force to the lesion or the like, which may include actual further radially outwardly directed movement of the balloon 26 beyond its working profile, as illustrated in phantom in Figure 1.

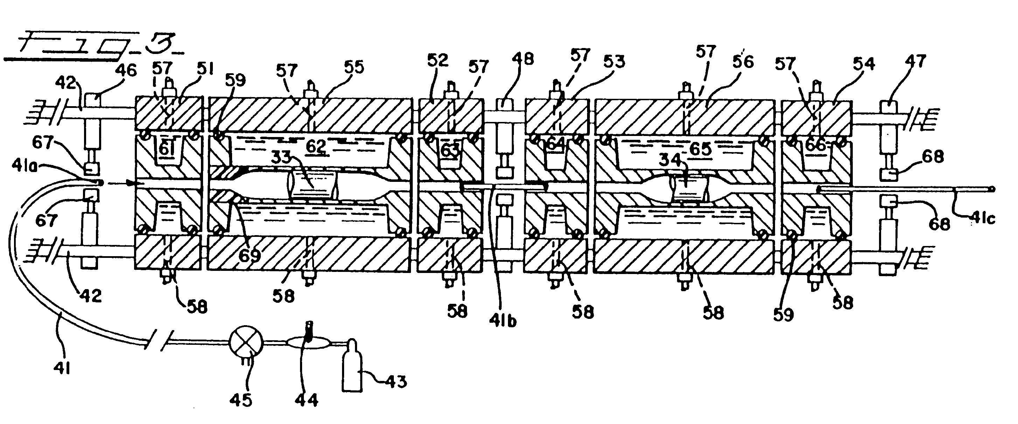

Figures 2a, 2b, 2c, 2d and 2e illustrate the transformation of a length of tubing 32 that is a material such as extruded nylon or polyamide into a balloon 33 or a modified balloon 34. Either balloon 33 or 34 possesses the ability to be first inflated to its unextended or working profile and then therebeyond to a limited and/or controlled extent by the application of greater pressure. Each balloon 33 and 34 includes a balloon portion 35, 36, respectively, and leg portions 37, 38, respectively. The leg portions 37, 38 are the portions of the balloon that are secured to the tube 22 of the catheter 21.

In an exemplary application, tubing length 32 is extruded so that it exhibits a diameter that is roughly one-quarter of the diameter intended for the balloon. The extruded tubing length 32 also has a nominal wall thickness that is on the order of six to twelve or so times the desired wall thickness of the balloon 33, 34.

Figure 2b illustrates tubing length 32a, which is tubing length 32, after it has been elongated to approximately three times its original length. This first step of elongation or drawing procedure is carried out at approximately room temperature, and same proceeds typically until it has been stretched to the point that it exhibits a noticeable resistance to further stretching. Typically, the pull force is greater than the yield point of the particular tubing length 32, but less than the ultimate tensile strength, lengthwise, of the selected material. Generally speaking, this axial elongation procedure is carried out until the wall thickness of the tubing length 32a is roughly one-half of the wall thickness of the tubing length 32 and/or until the diameter of the tubing length 32a is roughly one-half to forty percent of the outer diameter of the tubing length 32. The actual stretched length can typically be about two times to about four times or more of the original tubing length 32. Actual stretching of the tubing 32 can be performed by simply axially stretching length 32 or by pulling or drawing length 32 through a sizing die.

Figure 2c illustrates a second step that is carried out after the longitudinal elongation procedure of Figure 2b has been completed. This is a step by which a portion of the tubing length 32a expands primarily radially and thereby delineates the balloon portion 35 from the leg portions 37. This step is carried out by pressure exerted on the inside wall of the tubing length 32a by a pressurized fluid. Typically, the balloon portion 35 will have an outer diameter that is on the order of roughly six times the outer diameter of the tubing length 32a. The pressurized fluid may include gasses such as compound air, nitrogen or argon. Liquids such as water or alcohol could also be used if they do not pose a problem of leaving residual fluid in the balloon. Generally speaking, the larger the balloon, the faster will be the inflation fluid flow rate. Examples of cardiac balloons generally ranked in typical order of increasing size are those designed for use in coronary arteries, those designed for use in peripheral arteries, and valvuloplasty balloons for use in cardiac valves. Other balloons include uroplasty balloons for dilating the prostatic urethra.

The procedure illustrated in Figure 2c can be facilitated by controlling the location at which the radial expansion will occur. An advantageous manner of effecting this result is to carry out a local application of heat to the balloon portion 35 during radial expansion while avoiding such heat application at the leg portions 37. Elevating the temperature in such a localized area will lower the yield point of the nylon, polyamide or the like at that location and thereby facilitate the radial expansion of this selected area. As an example, a temperature for conducting this step typically can be between about 35° C. and perhaps as high as 90° C., the optimum temperature depending somewhat upon the particular material that is utilized.

Means are provided for controlling the expansion of the tubing length 32a and its formation into the balloon portion 35 and the legs 37. This can be accomplished by controlling and monitoring conditions and/or expansion positions within a molding apparatus. An exemplary means in this regard is included in the molding apparatus described herein. Otherwise, this function can be accomplished by closely controlling the rate of fluid passage and by monitoring the pressure thereof. For example, for a valvuloplasty balloon, the following approach can be taken. One end of the longitudinally stretched tube having a diameter of approximately 0.140 inch and a wall thickness of approximately 0.006 inch is sealed, and a liquid such as water is pumped into the tube at a rate of approximately 2 ml per minute. For tubing of this size and at a flow rate of this magnitude, balloon inflation begins at about 300 psi gauge and drops to around 150 psi gauge. Expansion continues in this manner until the wall of a mold bearing the desired shape of the balloon is encountered, which is observable by a significant pressure increase. Pumping continues until a pressure of about 180 to 200 psi is reached. This pressure condition can be held or maintained, if desired.

A satisfactory balloon can be prepared by proceeding with the method through and including the step illustrated in Figure 2c, followed by dimensional stabilization, heat setting or thermoforming, the balloon to near its bidirectionally stretched profile and size by maintaining its elevated temperature until the selected material is thermally set. Tailorability that is achieved according to this invention is a function of the particular heat setting conditions. The setting temperature can vary with the type of material used, the wall thickness, and the treatment time. A typical setting temperature is between about 100° C. and about 160° C. for a nylon material, and a typical time is from about 1 minute to about 4 minutes.

Such balloons 33 may include expansion knurls 39 that tend to appear in the areas generally extending between the legs 37 and the working surface of the balloon portion 35. These expansion knurls, which may be described as nodules, ridges, ribs, beads or the like, are believed to result from disproportionate expansion of the material such as nylon in this area. When it is desired to minimize the existence of these expansion knurls 39 on the balloon, secondary longitudinal stretching with radial shrinkage followed by secondary radial expansion can proceed with a balloon that is not thermally set, such being illustrated in Figure 2d and Figure 2e.

Regarding Figure 2d, the balloon 33 of Figure 2c, which is not thermally set, is again longitudinally oriented by applying an axially directed force that is typically greater than the yield point but less than the ultimate tensile strength of the balloon 33. If desired, the magnitude of this axial force can be substantially the same as the magnitude of the axial force applied in the procedure illustrated in Figure 2b. Further radial expansion is then conducted by, for example, introducing a pressurized fluid into the balloon lumen in order to prepare the balloon 34 shown in Figure 2e. It is often desirable to conduct this procedure within a mold cavity in order to thereby effect a careful shaping of the balloon portion 36, the leg portions 38, and the tapered connection surfaces therebetween, generally as desired. The resulting modified balloon 34 typically will not include any significant expansion knurls 39, and it will exhibit a uniform transition between the balloon portion 36 and the leg portions 38.

Whether the procedure is utilized that forms the balloon 33 or if the procedure is continued such that the balloon 34 is formed, the thus formed balloon 33, 34 is preferably then subjected to a heat setting step at which the still pressurized balloon 33, 34 is heated to set the expanded dimensions thereof. Setting will typically be accomplished at a temperature of 85° C. or greater, typically up to about the melting point of the balloon material, depending somewhat upon the actual material and the size and thickness of the balloon. For example, a typical heat setting temperature for Nylon 12 is 1 minute at 120° C. With this heat treatment, the balloon will retain this form and most of its expanded size, typically about 95% of more of it, upon being cooled. Preferably, the balloon remains pressurized until it is generally cooled. If this heat setting procedure is not utilized, the balloon will promptly shrink back to approximately 40% to 50% of the diameter to which it had been blown or biaxially oriented in the mold.

With more particular reference to this heat setting procedure, an important component in this regard is the use of a thermoformable material. A material is thermoformed if it can be formed to another shape. Nylon is a thermoforming plastic. It can be heated to a temperature below its melting point and deformed or formed to take on another shape and/or size. When cooled, thermoforming materials retain that new shape and/or size. This procedure is substantially repeatable. On the other hand, a thermosetting material, such as a natural latex rubber, a crosslinked polyethylene or a silicone rubber, once "set" is crosslinked into that size and/or shape and cannot be heat deformed. Also, some polyethylene terephthalates tend to crystallize and lose their usually inherent thermoforming ability. When a nylon balloon as discussed herein is heated for a prolonged period of time at temperatures greater than a predetermined elevated temperature, for example about 80° C., and then cooled, it becomes thermoformed to the balloon geometry. If the balloon were to be reheated to this temperature or above, it could be formed into a balloon having a different geometry.

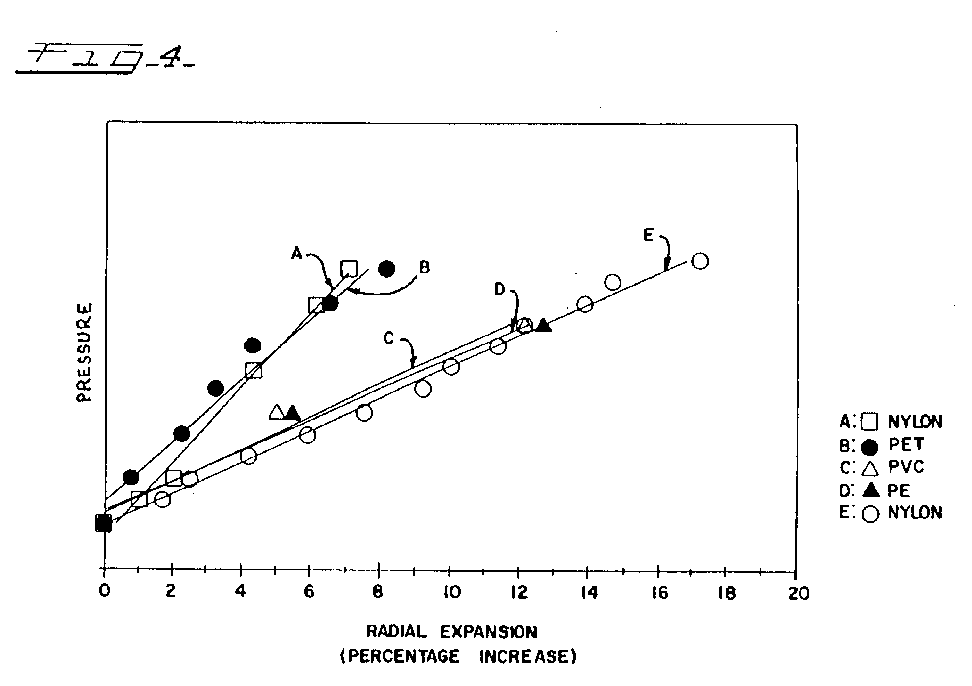

Figure 3 illustrates a molding apparatus that is suitable for fabricating medical balloons as discussed herein. The apparatus transforms a parison 41 into balloons for medical devices and the like. The apparatus achieves longitudinal stretching, radial expansion, heating and cooling, and it includes means for monitoring radial expansion, all of which can be conveniently controlled by suitable means such as hard circuitry, a microprocessor, or other computerized controlling arrangements. These various parameters that are controlled can thus be precisely set and easily modified in order to present the optimum conditions for fabricating a particular parison into a balloon having a specified sizing and the properties desired. Specific parameter values are presented herein which are typically suitable for balloon materials such as nylons, and it will be understood that these parameter values can be modified as needed for the specific material being shaped into the balloon.

The apparatus that is illustrated also has the ability to generally simultaneously perform different steps on multiple balloon portions of the parison 41 as it passes through the apparatus. This can be especially useful because of variations in wall thickness and other attributes of each batch of tubing that is used as the parison 41.

The apparatus includes a series of components which are suitably mounted with respect to each other, such as along slide rails 42. From time to time, movement of some of the components along the rails or the like is carried out by suitable means, such as piston assemblies (not shown) in accordance with generally known principles and structures.

A pressurized fluid supply 43 is provided to direct pressurized fluid into an end portion of the parison 41, typically in conjunction with one or more regulators 44 in accordance with well-known principles. One or more valves 45 also assist in controlling the flow rate and volume of pressurized fluid within the parison 41. Gripper assemblies 46 and 47 are provided for engaging different locations of the parison 41. An intermediate gripper assembly 48 is preferably also provided. One or more chiller chambers 51, 52, 53, 54 are also preferably provided. These are particularly useful as means for thermally isolating assemblies of the apparatus from each other. Also included are a free-blow chamber or mold 55 in which mechanical stretching of the parison 41 in two directions can be achieved and a molding chamber 56.

Preferably, means are provided by which the temperature of these various chambers is controlled and/or varied, for example between about 0° C. and about 150° C. or more. The illustrated assembly in this regard includes a jacket for confining a thermal fluid that is pumped thereinto and out thereof by suitable fluid inlets 57 and fluid outlets 58. O-rings 59 or the like are provided in order to contain the thermal fluid within each fluid jacket 61, 62, 63, 64, 65, 66.

In using the illustrated apparatus, the parison 41 is initially fed or threaded through the entire apparatus, typically between gripper assembly 46 and gripper assembly 47. Gripping pads 68 of the gripper assembly 47 move inwardly and engage the downstream end portion of the parison 41 to the extent that it pinches off this portion of the parison 41, preferably also heat sealing the parison at this time. Generally simultaneously, gripping pads 67 of the gripper assembly 46 engage the parison 41 at the illustrated upstream location of the apparatus to such an extent that the gripping pads 67 will prevent movement of the parison 41 with respect to the gripping pads 67, but still permit the flow of pressurized fluid thereacross when desired. Gripper assembly 47 then moves in a downstream direction (to the right as illustrated in Figure 3) until the length of the parison that is secured between gripper assemblies 46 and 47 is stretched longitudinally to in excess of twice its length up to as great as about four times its length or more, a typical stretching being approximately three times this unstretched length.

Next, the free-blow radial expansion chamber 55 is heated by passing heated thermal fluid into the fluid jacket 62. The chiller chambers 51, 52, or other suitable means, provide a thermal variant such that the heat from the fluid jacket 62 is imparted only to that length of the parison that is substantially within the free-blow chamber 55. The temperature of this particular portion of the parison 41 will be heated to a temperature of roughly between about 70° C. and 120° C. or more, depending upon the particular parison 41 and the balloon properties desired. At this time, pressurized fluid within the parison 41 that originates from the supply 43 passes through the parison length at the gripper assembly 46 and into the parison length at the free-blow chamber 55. If desired, the gripper assembly 48 can be utilized in order to confine this particular pressure application to the section of the parison 41 that is upstream thereof.

A primary objective of the free-blow chamber 55 is to radially expand that portion of the parison 41 into a balloon 33 as generally illustrated in Figure 2c. It is often desired to 5 precisely control the amount of radial expansion, and means in this regard are provided. The means illustrated in Figure 3 includes a slidable insert or ring 69. When this insert 69 is engaged by the expanding balloon 33, it moves against an air or metallic spring (not shown) in a direction to the left as illustrated in Figure 3 until it trips a suitable switch or the like (not shown), which signals that the desired degree of radial expansion has been achieved. At this time, steps are taken to interrupt the radial expansion. This typically includes cooling by exchanging the heated thermal fluid within the fluid jacket 62 for thermal fluid having a colder temperature, typically on the order of about 10° C. or somewhat above or below depending upon the particular parison 41 and the particular properties desired.

The pressure imparted to balloon portion 33 is then depleted by, for example, permitting exhaustion thereof through the valve 45 after the balloon has been cooled. If utilized, the gripper assembly 48 is released, and the balloon portion 43 is moved from the free-blow chamber 55 into the molding chamber 56 by movement of the downstream end portion of the parison by the gripper assembly 47. This typically simultaneously accomplishes the longitudinal orientation stage depicted in Figure 2d. Once this positioning takes place, the pressurized fluid is pumped into that portion of the parison that is within the molding chamber 56, and thermal fluid is passed into the fluid jacket 65 in order to heat this portion of the parison to an elevated temperature, again between about 70° C. and up to just below the melting point, for example 150° C. or more, depending upon the particular parison and the properties desired of the balloon.

Generally speaking, it is usually advantageous that the temperature in the molding chamber 56 be higher than that applied in the free-blow chamber 55, while at the same time imparting a pressure to the inside walls of the parison within the molding chamber 56 that is equal to or lower than the pressure applied in the free-blow chamber 55. For example, when a nylon is the material, the temperature in the free-blow chamber 55 can be slightly above ambient, preferably in a range of between about 30° C. and 60° C., while the temperature in the molding chamber 56 can be at the high end of this range or even well above, as needed. Exemplary pressures would include on the order of ten atmospheres in the molding chamber 56 and twice that pressure or greater in the free-blow chamber 55. The exact pressure is determined by the material and by the wall thickness and hoop stress of the balloon to be molded.

With heat thus imparted to the modified balloon 34 within the molding chamber 56, the balloon 34 is thereby thermoformed, with heat setting in this regard involving raising the temperature of the thermoplastic while it is under inflated stress. Thereafter, the heated fluid within the fluid jacket 65 is exchanged for cooling fluid in order to substantially maintain the size and shape of the balloon 34 formed within the molding chamber 56. After the pressure has been relieved, the balloon is removed from the apparatus. Subsequently, the thus modified parison is severed generally along lines A and B as illustrated in Figure 2e in order to thereby form the balloon 26 for inclusion within a medical device such as the catheter 21.

It will be appreciated that the parison will include balloons in various stages of their formation as the parison passes through the apparatus. Preferably, this is accomplished in a somewhat reverse manner, as follows. After initial stretching and formation of a balloon 33 within the free-blow chamber 55, the apparatus is utilized so that the portion of the parison within the molding chamber 56 is radially expanded before that within the free-blow chamber 55, which generally occurs as follows. Molding chamber 56 is heated as described herein and pressurized to form the balloon 34. Thereafter, the gripper assembly 48 closes off the parison between the chambers 55 and 56. The free-blow chamber 55 is then heated, and the radial expansion is carried out as described herein in order to form the balloon 33. Balloon 33 is then moved into the molding chamber 56, and the process is essentially repeated.

It should be appreciated that the balloon can be made entirely in the mold section 56 without free blowing in compartment 55. However, balloons made in this manner may demonstrate knurling.

Regarding the fluids suitable for use in the apparatus, it is typically advantageous to have the pressure source 43 provide a fluid that does not require excessive treatment to remove same from the internal surfaces of the finished medical device balloon 26. To be taken into consideration in this regard are moisture content of fluids and ease and safety of disposal of the fluid. A particularly suitable fluid is pressurized nitrogen gas. With respect to the fluid for use within the various fluid jackets 61 through 66, it is typically best to utilize a fluid that is in its liquid state throughout whatever processing temperatures might be needed for the apparatus. Preferably the fluid is one that will likewise maintain a generally consistent viscosity throughout the processing range, for example, avoiding the onset of solidification or crystal formation or the like that would modify the properties of the thermal fluid at lower processing temperatures.

With respect to the materials out of which the balloon and parison are made, they are typically nylons or polyamides. Preferably, these materials have an amorphous nature while still possessing enough crystallinity to be processed under the conditions provided according to this invention. The materials should also have substantial tensile strength, be resistant to pin-holing even after folding and unfolding, and be generally scratch resistant. The material should have an intrinsic viscosity that enables blowing at elevated temperatures. A typical intrinsic viscosity range that is preferred for the nylon or polyamide materials according to this invention is between about 0.8 and about 1.5, with about 1.3 being especially preferred. It is also desired that the material have a reasonably good moisture resistance. Materials of the Nylon 12 type have been found to possess these advantageous properties. Other exemplary nylons include Nylon 11, Nylon 9, Nylon 69 and Nylon 66.

With more particular reference to the intrinsic viscosity of nylon or polyamide materials, it is believed that such materials are able to generally maintain their intrinsic viscosities during extrusion and through to final balloon fabrication. It is further understood that other materials such as polyethylene terephthalate which have been used to fabricate balloons for medical devices typically do not exhibit this maintenance of intrinsic viscosity, but the intrinsic viscosity of the pellet material drops substantially during extrusion, with the result that a biaxially oriented polyethylene terephthalate balloon is understood to have an intrinsic viscosity which is lower than that of the polyethylene terephthalate pellets from which it originated. It is further believed that a higher balloon intrinsic viscosity reduces the likelihood that pin-holing will develop in the finally fabricated balloon.

With further reference to the nylon or polyamide materials which may be used according to this invention, they are bondable to the catheter 21 by epoxy adhesives, urethane adhesives, cyanoacrylates, and other adhesives suitable for bonding nylon or the like, as well as by hot melt bonding, ultrasonic welding, heat fusion and the like. Furthermore, these balloons may be attached to the catheter by mechanical means such as swage locks, crimp fittings, threads and the like. Nylon or polyamide materials can also be provided in the form in which they are reinforced with linear materials such as Kevlar and ultra high tensile strength polyethylenes. The polymer materials used can also be coated with pharmaceutical materials such as heparin and the like, non-thrombogenic lubricants such as polyvinyl pyrrolidone and the like. Additionally, the polymer materials can be filled with radiopaque media such as barium sulfate, bismuth subcarbonate, iodine containing molecules, tungsten, or other fillers such as plasticizers, extrusion lubricants, pigments, antioxidants and the like.

Nylons or polyamides as used according to the present invention are flexible enough to tolerate a greater wall thickness, even in the leg areas, while still providing a structure that is foldable onto itself for insertion into a body cavity or guiding catheter. These nylons or polyamides, when formed into balloons as discussed herein, have a calculated tensile strength of between about 15,000 and about 35,000 psi and above, preferably between about 20,000 and about 32,000 psi.

The materials according to the present invention form medical device balloons that exhibit the ability to be expanded first to a non-stretched or non-distended condition, or working size, upon the application of a given pressure. They also have the ability to be inflated further so as to be stretched therebeyond in a controlled and limited manner. The degree of such stretching or expansion can, within limits, be tailored as needed. In other words, the balloons according to the present invention allow for some growth at a pressure higher than that needed to merely fill the balloon, but this additional growth or expansion is not so substantial that there is an overinflation danger.

Materials according to this invention should be able to be tailored during balloon formation to possess the ability to be stretched a generally predetermined percentage beyond its non-distended or working diameter, the amount of this percentage depending upon conditions under which the parison was processed into the balloon. A suitable material according to the present invention can be tailored to cover values within a span of at least 10 percentage points of radial expansion. Certain materials including some nylons can exhibit a tailorability range of 25 or 30 percentage points or more and can, for example, exhibit a radial expansion of 10 percent or below and as high as about 30 percent or above.

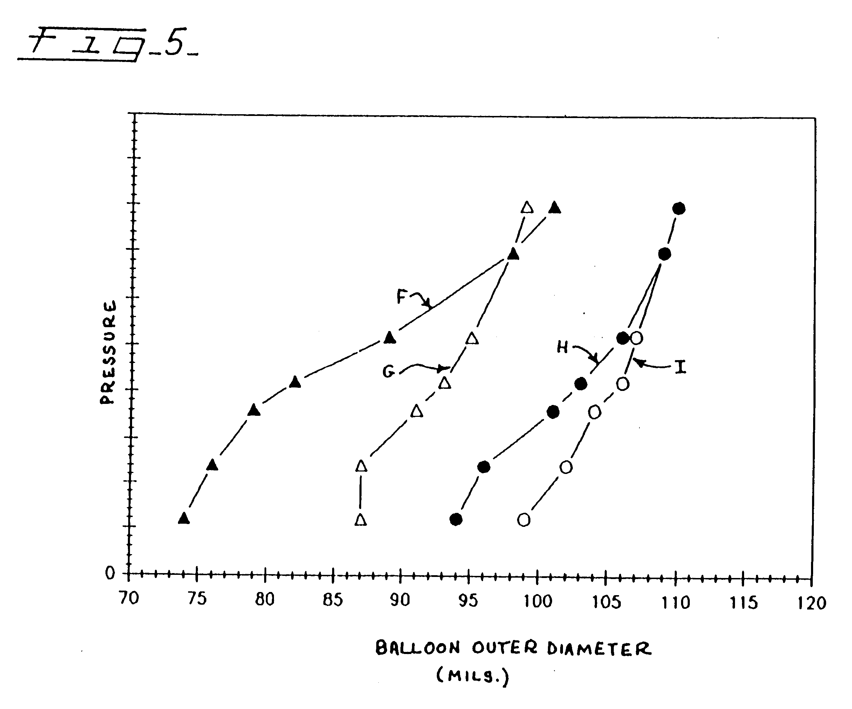

In order to illustrate this situation, tests were run on angioplasty balloons of different materials, namely polyvinyl chloride, cross-linked polyethylene, polyethylene terephthalate and nylon. These data are reported graphically in Figure 4. Wall thicknesses varied depending upon the properties of the material in order to provide a viable balloon. The polyethylene terephthalate balloon (B) had a wall thickness of 0.010 mm. The polyvinyl chloride balloon (C) had a wall thickness of 0.064 mm, and the polyethylene balloon (D) had a wall thickness of 0.058 mm. (Data for balloons C and D are as reported in "Effects of Inflation Pressure on Coronary Angioplasty Balloons," The American Journal of Cardiology, January 1, 1986, these tests being run under ambient conditions, while the remaining balloons in Figure 4 were tested at 37° C.) One of the nylon balloons (A) had a wall thickness of 0.013 mm and a hoop expansion ratio of 5.2. Another of the nylon balloons (E) had a wall thickness of 0.008 mm and a hoop expansion ratio of 4.3. Tailorability of balloons according to this invention is a function of hoop expansion ratio, which is defined as mean balloon diameter divided by mean as-extruded tubing or parison diameter.

Figure 4 plots the relative pressure of the fluid imparted to the various angiographic catheters against the percentage increase in size (such as diameter or radius) of the balloon beyond its working size, designated as "O." It will be noted that the more compliant materials, such as polyvinyl chloride, gradually grow with increased pressure and permit some limited additional pressure increase after the working size has been achieved and before further expansion ceases prior to reaching the burst limit of the balloon. It will be appreciated that, with materials such as these, a smaller increase (when compared with materials of plots A and B) in relative pressure significantly increases the balloon size. Materials such as polyethylene terephthalate (PET) inflate to their working size, but not therebeyond to an extent as great as others of the plotted curves.

The nylon or polyamide material illustrated by curve E exhibits a relatively long and flat curve in Figure 4, which indicates that it will continue to expand moderately upon the application of relatively small additional increments of inflation pressure. Once the unstretched or non-distended profile or working size is reached, this tailored nylon balloon possesses adequate stretchability or compliance to the extent that it can ramp up to another inflated profile. While the polyethylene balloon of curve D initially distends to about the same extent as does the nylon curve E balloon, this nylon balloon can tolerate greater inflation pressures without bursting than can the curve D polyethylene balloon or the curve C polyvinyl chloride balloon. This observation is especially significant when it is appreciated that the curve E nylon balloon had a substantially thinner wall diameter. Based upon data such as that illustrated in Figure 4, it is possible to predict the compliant expansion associated with added inflation pressure. A balloon in accordance with the present invention can exhibit this growth property so that it will radially expand from a low of about 5 percent beyond the working size to a high of about 35 percent or more of the working size.

As previously observed herein, balloon expansion tailorability is a function of heat setting conditions and of hoop expansion ratio for balloon materials according to the present invention. For such materials, increasing the heat set temperature increases the size of the finished balloon at a given inflation pressure and decreases the shrinkage which typically occurs after sterilization such as a standard ethylene oxide treatment. Also, the amount of hoop expansion imparted to the parison during processing has a significant effect on the amount of distention which the finished balloon will exhibit.

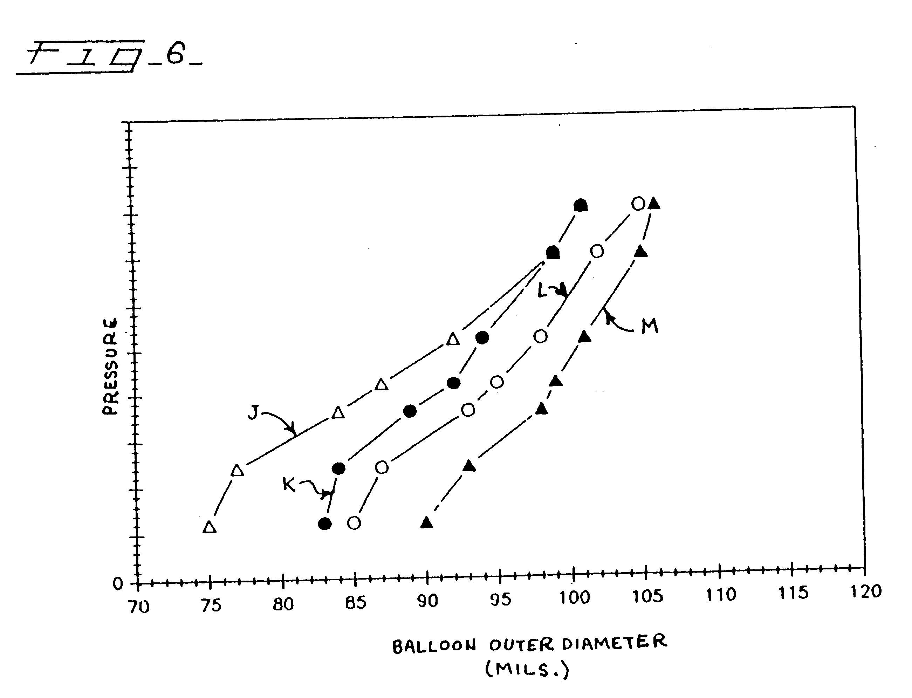

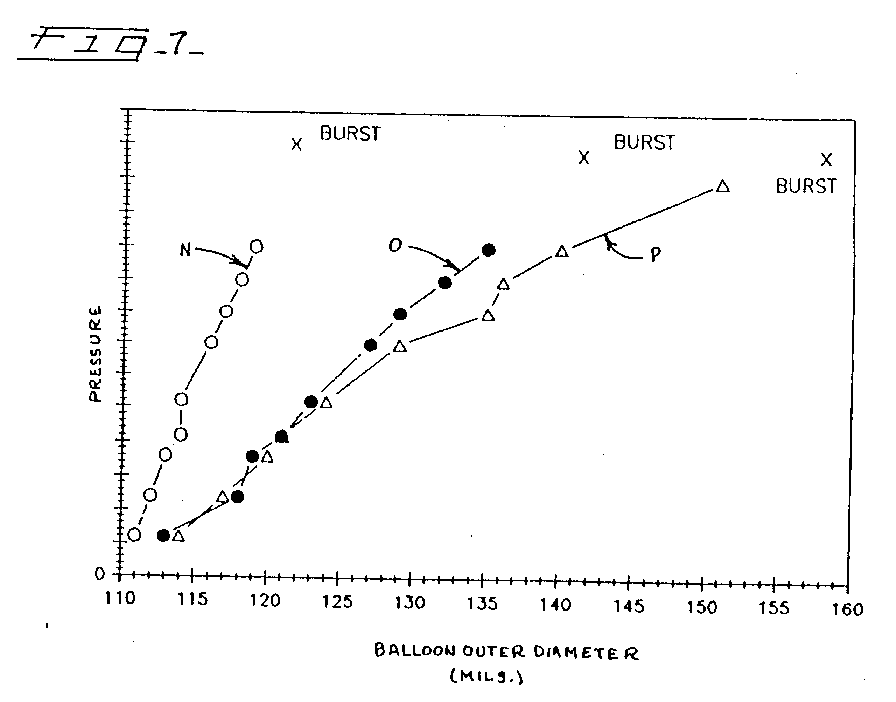

Figure 5 and 6 illustrate the effect of varying heat setting conditions on the distensibility of the finished balloon. This heat setting is achieved when the balloon is subjected to a temperature greater than the glass transition temperature of the balloon material and is allowed to cool to below the glass transition temperature. For a material such as a nylon or a polyamide, temperatures between about 90° C. and about 180° C. have a pronounced heat setting capability. The glass transition temperatures may be less than this heat setting temperature and may include temperatures in the range of 20° C. to about 40° C., and the balloon will exhibit good flexibility at body temperature which can, for example, facilitate balloon insertion and maneuverability during use.

Figure 5 gives four distention plots, tested at 37° C. for nylon balloons that are substantially identical except they were subjected to different heat set temperatures. In each case, the balloon was subjected to a 2 minute heat/cool cycle, with cooling being to room temperature. Each balloon was subsequently subjected to ethylene oxide sterilization. Curve F was subjected to a heat set temperature of 120° C., curve G was at 130° C., curve H was at 140° C., and curve I was at 150° C. It will be noted that the curves illustrate different distention properties and the type of tailoring thus achieved. Figure 6 provides a similar illustration and further shows an effect of the ethylene oxide sterilization. While curves J and K both are for balloons which were heat set at 100° C., the curve J balloon was sterilized while the curve K balloon was not. Likewise, the balloons of curves L and M both were heat set at 140° C., curve L illustrating a sterilized situation, and curve M a non-sterilized situation.

The relationship between balloon tailorability and hoop expansion ratio is illustrated in Figure 7. Three non-sterilized nylon balloons having different hoop expansion ratios were tested at 37° C., the expansion being to burst. A relatively high hoop expansion ratio of 4.9 (curve N) gave a balloon distention (safely short of burst) of about 7 percent. A 3.7 hoop expansion ratio (curve O) gave a balloon distention of about 23 percent, while a 3.3 hoop expansion ratio (curve P) gave a balloon distention of about 36 percent.

Furthermore, nylon or polyamide balloons according to the present invention provide controlled expansion properties and tolerate given pressures without bursting without requiring wall thicknesses as great as those needed for other materials such as polyvinyl chloride or polyethylene. This thin wall thickness of these nylon balloons permits the balloon to enter smaller vessels, lesions and/or catheters. Suitable balloons according to this invention can have shell or wall thicknesses as low as about 0.0002 inch and as high as 0.002 inch. An exemplary preferred shell thickness range is between about 0.0004 and about 0.001 inch, or between about 0.0005 and about 0.0008 inch.

A material such as nylon is softer and more flexible at body temperature, the temperature at which it is used, than other balloon materials such as polyethylene terephthalate which have a glass transition temperature far in excess of body temperature. For at least some nylons, the glass transition temperature approximates body temperature, and nylon balloons generally exhibit amorphous properties that enhance flexibility and softness even after proceeding with the type of processing described herein, including thermoforming and the like. Nylon balloons thus can be at a glass transition state while they are within the body, which is not the case for numerous other materials that can be used as medical balloons.

Balloons made of nylons exhibit a property such that, when they burst, the diameter of the balloon will decrease, thereby facilitating removal from the body. It is believed this decrease may be caused by a relaxing of the material which noticeably reduces the size of the balloon. Other materials such as polyethylene terephthalate are not known to exhibit this advantageous property. A disadvantageous property that can be characteristic of polyethylene terephthalate medical balloons is the development of extensive material wrinkling upon sterilization, which wrinkles persist, even at body temperature, until very high inflation pressures are applied, on the order of 200 psi. When a nylon or polyamide balloon is sterilized, few if any material wrinkles develop, and these substantially disappear at relatively low inflation pressures, on the order of 8 psi.

Nylon materials have been observed to exhibit desirable stability during processing to the extent that they do not absorb excessive moisture from the environment if the parison is allowed to stand uncovered for reasonable time periods. Materials such as polyethylene terephthalate are degraded by environment moisture if the parison is not stored in protective bags or the like prior to bidirectional stretching.

It will be understood that the embodiments of the present invention which have been described are illustrative of some of the applications of the principles of the present invention. Numerous modifications may be made by those skilled in the art without departing from the scope of the claims.

## Claims

1. A molding apparatus for fabricating medical device balloons, the apparatus including a blow molding chamber (56) having an infeed opening and an outfeed opening, means for feeding a parison (41) through the infeed opening and outfeed opening of said blow molding chamber (56), means for axially elongating (46, 67, 47, 68) said parison in a first direction while a first portion of said parison is within said blow molding chamber (56), means for supplying pressurized fluid (43, 44, 45) into said parison (41) and for effecting radial enlargement of said first portion of the parison (41) within said blow molding chamber (56) upon passage of pressurized fluid from the supplying means (43, 44, 45) into said first portion of the parison,
**characterized by**
a free-blow chamber (55) having an infeed opening and an outfeed opening upstream of said blow molding chamber (56),
said means for feeding the parison (41) also feeds same through the infeed opening and the outfeed opening of said free-blow chamber (55),
said means for axially elongating (46, 67, 47, 68) said parison in a first direction also stretches said parison while a second portion thereof is within said free-blow chamber (55),
said means for supplying pressurized fluid (43, 44, 45) supplies same at a location upstream of said infeed opening of the free-blow chamber (55),
said free-blow chamber (55) has means for permitting stretching of a defined portion of said parison in a second direction by radially enlarging said second portion of the parison (41) therewithin upon passage thereinto of pressurized fluid from said supplying means (43, 44, 45), and
means for thermally isolating (51, 52, 53, 54) said free-blow chamber (55) from other components of the apparatus including said blow molding chamber (56).

2. The molding apparatus according to claim 1, further including heat transfer means (62) for raising the temperature of the second portion of the parison (41) within said free-blow chamber (55) during said radial enlargement of this second portion of the parison (41) in order to form a radially expanded portion of the parison (41).

3. The molding apparatus according to claim 1 or 2, wherein heat transfer means (62, 63, 64, 65) lowers the temperature of said radially expanded parison portion such that said axially elongating means (46, 67, 47, 68) axially orients the parison (41) when same is at room temperature or less.

4. The molding apparatus according to any one of claims 1 - 3, wherein said parison (41) is a nylon or a polyamide and said axially elongating means (46, 67, 47, 68) axially elongates said parison having a temperature of room temperature or below to at least in excess of twice the length of the parison (41) prior to said axial elongation.

5. The molding apparatus according to any one of claims 1 - 4, further including heat transfer means (65) for raising the temperature of the first portion of the parison (41) within said blow molding chamber (56) during said radial enlargement of this first portion of the parison (41) and for subsequently cooling said portion of the parison (41).

6. The molding apparatus according to claim 5, wherein said heat transfer means imparts a temperature between 0° C and 150° C to said blow molding chamber (56).

7. The molding apparatus according to any one of claims 1 - 6, wherein said means for thermally isolating (51, 52, 53, 54) said chambers (55, 56) include chiller chambers substantially juxtaposed to the infeed and outfeed openings of said free-blow chamber (55) and of said blow molding chamber (56).

8. The molding apparatus according to any one of claims 1 - 7, wherein said axially elongating means (46, 67, 47, 68) includes upstream gripping means (46, 67) for preventing longitudinal movement of the parison (41) and downstream gripping means (47, 68) for longitudinally translating the parison and for substantially preventing passage of the pressurized fluid thereby, said upstream gripping means (46, 67) being upstream of said free-blow chamber (55), and said downstream gripping means (47, 68) being downstream of said blow molding chamber (56).

9. The molding apparatus according to claim 8, further including intermediate gripping means (48) for substantially preventing passage of the pressurized fluid within the parison (41) between said free-blow chamber (55) and said blow molding chamber (56).

10. The molding apparatus according to any one of claims 1 - 9, further including means (69) for limiting the amount of parison radial expansion that is effected by said free-blow chamber (55).

11. The molding apparatus according to claim 10, wherein said expansion limiting means includes a member (69) that is activated upon being engaged at a preselected location by said parison (41) while it is expanding within said free-blow chamber (55).

12. The molding apparatus according to claim 11, wherein said member (69) of the controlling means activates heat transfer means for lowering the temperature of the parison within the free-blow chamber (55) from a free-blow temperature of 75° C to as low as room temperature.

13. A process for fabricating medical device balloons, the process including the steps of:
feeding (Fig. 2a) an elongated parison (41) through a molding apparatus including a blow molding chamber (56) in which mechanically stretching of said parison in two directions can be achieved;
axially stretching (Fig. 2b) to longitudinally elongate the parison within said blow molding chamber (56) to provide an axially elongated parison length;
radially expanding (Fig. 2c) said axially elongated parison length within said blow molding chamber (56) by infusing pressurized fluid thereinto to provide a radially expanded parison length;
**characterized in that**
said feeding step (Fig. 2a) includes feeding the parison (41) through a free-blow chamber (55) upstream of the blow molding chamber (56);
axially stretching (Fig. 2b) said parison (41) within said free-blow chamber (55) to longitudinally elongate said parison (41) within said free-blow chamber (55);
blow molding (Figs. 2c, 3) said axially stretched parison length by infusing pressurized fluid thereinto while same is within the free-blow chamber (55) to provide, together with said feeding, axially stretching and radially expanding steps, a molded parison in the form of a medical device balloon.

14. The process according to claim 13, wherein said axially stretching steps (Fig. 2b) within said free-below chamber (55) and blow molding chamber (56) are carried out at room temperature.

15. The process according to claims 13 or 14, wherein said radially expanding step (Fig. 2c) is carried out at an elevated temperature of between 80° C and 150° C.

16. The process according to any one of claims 13 - 15, wherein said blow molding step (Figs. 2c, 3) is carried out at an elevated temperature of between 80° C and 150° C.

17. The process according to any one of claims 13 - 16, wherein said feeding step (Fig. 2a) feeds a parison tube of a nylon or of a polyamide, and wherein said step of axially stretching (Fig. 2b) the fed parison elongates the parison to at least twice the length of the parison prior to the axially stretching step.

18. The process according to any one of claims 13 - 17, further including interposing a thermal isolating means (52, 53) between the free-blow chamber (55) and the blow molding chamber (56) so that the elevated temperature of the radially expanding step is substantially confined to the portion of the parison to be radially expanded.

19. The process according to claims 13 - 18, wherein said step of axially stretching within said blow molding chamber (56) includes axially stretching (Fig. 2d) said molded parison until its diameter is decreased and blow molding (Fig. 2e) said axially stretched molded parison.

20. The process according to any one of claims 13 - 19, further including controlling the extent of radial expansion achieved by said blow molding step (Figs. 2c, 3) by carrying out the blow molding at an elevated temperature and then reducing the temperature within the free-blow chamber (55) to room temperature or below as soon as a desired extent of radial expansion is achieved.

21. The process acccording to any one of claims 19 - 20, further including raising the temperature of the radially expanded and axially stretched parison within the blow molding chamber (56) and infusing the pressurized fluid into a portion of the elongated parison that is upstream of the free-blow chamber (55) whereby the pressurized fluid flows through the parison within the free-blow chamber (55) and then into the radially expanded and axially stretched parison within the blow molding chamber (56); and closing off the parison between the free-blow chamber (55) and the blow molding chamber (56) to prevent passage of pressurized fluid therebetween.

## Patentansprüche

1. Eine Formvorrichtung zum Herstellen von Ballonen für ein medizinisches Gerät, wobei die Vorrichtung einschließt: eine Blasformkammer (56) mit einer Zuführöffnung und einer Ausführöffnung, Mittel zum Zuführen eines Vorformlings (41) durch die Zuführöffnung und Ausführöffnung der Blasformkammer (56), Mittel zum axialen Dehnen (46, 67, 47, 68) des Vorformlings in einer ersten Richtung während ein erster Abschnitt des Vorformlings sich innerhalb der Blasformkammer (56) befindet, Mittel zum Zuliefern eines unter Druck stehenden Fluides (43, 44, 45) in den Vorformling (41) und zum Bewirken einer radialen Vergrößerung des ersten Abschnittes des Vorformlings (41) innerhalb der Blasformkammer (56) nach Passage des unter Druck stehenden Fluides aus den Zuliefermitteln (43, 44, 45) in den ersten Abschnitt des Vorformlings,
**gekennzeichnet durch**
eine Freiblaskammer (55) mit einer Zuführöffnung und einer Ausführöffnung stromaufwärts von der Blasformkammer (56),
wobei die Mittel zum Zuführen des Vorformlings (41) denselben ebenfalls durch die Zuführöffnung und die Ausführöffnung der Freiblaskammer ((55) führen,
wobei die Mittel zum axialen Dehnen (46, 67, 47, 68) des Vorformlings in einer ersten Richtung den Vorformling ebenfalls ausdehnen, während sich dessen zweiter Abschnitt innerhalb der Freiblaskammer (55) befindet,
wobei die Mittel zum Liefern des unter Druck stehenden Fluides (43, 44, 45) dasselbe an einem Ort stromaufwärts der Einführöffnung der Freiblaskammer (55) liefert,
wobei die Freiblaskammer (55) Mittel aufweist, welche es erlauben, einen definierten Abschnitt des Vorformlings in einer zweiten Richtung durch radiales Vergrößern des zweiten Abschnittes des Vorformlings (41) darin auszudehnen, nach Passage von unter Druck stehendem Fluid hierein aus den Zuliefermitteln (43, 44, 45), und
Mittel zum Wärmeisolieren (51, 52, 53, 54) der Freiblaskammer (55) von anderen Komponenten der Vorrichtung, die Blasformkammer (56) einschließend.

2. Die Formvorrichtung gemäß Anspruch 1, des weiteren einschließend Wärmeübertragungsmittel (62) zum Erhöhen der Temperatur des zweiten Abschnittes des Vorformlings (41) innerhalb der Freiblaskammer (55) während der Radialvergrößerung dieses zweiten Abschnittes des Vorformlings (41), um einen radial expandierten Abschnitt des Vorformlings (41) zu bilden.

3. Die Formvorrichtung nach Anspruch 1 oder 2, worin das Wärmeübertragungsmittel (62, 63, 64, 65) die Temperatur des radial expandierten Vorformlingabschnittes derart senkt, daß das axial dehnende Mittel (46, 67, 47, 68) den Vorformling (41) axial orientiert, wenn sich derselbe bei Raumtemperatur oder darunter befindet.

4. Die Formvorrichtung nach irgendeinem der Ansprüche 1-3, worin der Vorformling (41) ein Nylon- oder ein Polyamid ist und das axial dehnende Mittel (46, 67, 47, 68) den Vorformling, welcher eine Temperatur von Raumtemperatur oder darunter aufweist auf wenigstens mehr als das Zweifache der Länge des Vorformlings (41) vor der axiallen Dehnung dehnt.

5. Die Formvorrichtung nach irgendeinem der Ansprüche 1-4, ferner einschließend Wärmeübertragungsmittel (65) zum Erhöhen der Temperatur des ersten Abschnittes des Vorformlings (41) innerhalb der Blasformkammer (56) während der radialen Vergrößerung dieses ersten Abschnittes des Vorformlings (41) und zum nachfolgenden Kühlen des Abschnittes des Vorformlings (41).

6. Die Formvorrichtung nach Anspruch 5, worin das Wärmeübertragungsmittel der Blasformkammer (56) eine Temperatur zwischen 0°C und 150°C verleiht.

7. Die Formvorrichtung nach irgendeinem der Ansprüche 1-6, worin das Mittel zum Wärmeisolieren (51, 52, 53, 54) der Kammern (55, 56) Kühlkammern einschließt, welche im wesentlichen den Zuführ- und Ausführöffnungen der Freiblaskammer (55) und der Blasformkammer (56) benachbart sind.

8. Die Formvorrichtung nach irgendeinem der Ansprüche 1-7, worin die axial dehnenden Mittel (46, 67, 47, 68) Stromaufwärts-Greifmittel (46, 67) einschließt zum Verhindern der Längsbewegung des Vorformlings (41) und Stromabwärts-Greifmittel (47, 68) zum longitudinalen Verschieben des Vorformlings und zum im wesentlichen Verhindern der Passage von dem unter Druck stehenden Fluid dadurch, wobei die Stromaufwärts-Griffmittel (46, 67) stromaufwärts der Freiblaskammer (55) gelegen sind, und wobei die Stromabwärts-Greifmittel (47, 68) stromabwärts von der Blasformkammer (56) gelegen sind.

9. Die Formvorrichtung gemäß Anspruch 8, ferner einschließend Zwischengreifmittel (48) zum im wesentlichen Verhindern der Passage des unter Druck stehenden Fluides innerhalb des Vorformlings (41) zwischen der Freiblaskammer (55) und der Blasformkammer (56).

10. Die Formvorrichtung gemäß irgendeinem der Ansprüche 1-9, ferner einschließend Mittel (69) zum Begrenzen des Betrages der radialen Expansion des Vorformlings, welche durch die Freiblaskammer (55) bewirkt wird.

11. Die Formvorrichtung nach Anspruch 10, worin die Expansionsbegrenzungsmittel ein Teil (69) einschließen, welches aktiviert wird, nachdem es an einem vorher ausgewählten Ort durch den Vorformling (41) eingegriffen wird, während es sich innerhalb der Freiblaskammer (55) ausdehnt.

12. Die Formvorrichtung nach Anspruch 11, worin das Teil (69) der Steuermittel die Wärmeübertragungsmittel aktiviert zum Senken der Temperatur des Vorformlings innerhalb der Freiblaskammer (55) von einer Freiblastemperatur von 75°C bis so niedrig wie Raumtemperatur.

13. Ein Verfahren zum Herstellen von Ballons für medizinische Vorrichtungen, wobei das Verfahren die Schritte einschließt:
Zuführen (Fig. 2a) eines gedehnten Vorformlings (41) durch eine Formvorrichtung, welche eine Blasformkammer (56) einschließt, in welcher mechanisches Ausdehnen des Vorformlings in zwei Richtungen erreicht werden kann;
axiales Ausdehnen (Fig. 2b), um den Vorformling innerhalb der Freiblaskammer (56) longitudinal zu dehnen, um eine axial gedehnte Vorformlingslänge zur Verfügung zu stellen;
radiales Expandieren (Fig. 2c) der axial gedehnten Vorformlingslänge innerhalb der Blasformkammer (56) durch Infundieren eines unter Druck stehenden Fluides hierein, um eine radial expandierte Vorformlingslänge zur Verfügung zu stellen;
dadurch gekennzeichnet, daß
der Zuführschritt (Fig. 2a) das Zuführen des Vorformlings (41) durch eine Freiblaskammer (55) stromaufwärts von der Blasformkammer (56) einschließt;
axiales Ausdehnen (Fig. 2b) des Vorformlings (41) innerhalb der Freiblaskammer (55), um den Vorformling (41) longitudinal innerhalb der Freiblaskammer (55) zu dehnen;
Blasformen (Figuren 2c, 3) der axial ausgedehnten Vorformlingslänge durch Infundieren eines unter Druck stehenden Fluides hierein, während dieselbe sich innerhalb der Freiblaskammer (55) befindet, um zusammen mit den Zuführ-, axialen Ausdehnungs- und radialen Expansionsschritten einen geformten Vorformling in der Form eines Ballons für eine medizinische Vorrichtung zur Verfügung zu stellen.

14. Das Verfahren gemäß Anspruch 13, wobei die axialen Ausdehnungsschritte (Fig. 2b) innerhalb der Freiblaskammer (55) und Blasformkammer (56) bei Raumtemperatur durchgeführt werden.

15. Das Verfahren gemäß den Ansprüchen 13 oder 14, wobei der radiale Ausdehnungsschritt (Fig. 2c) bei einer erhöhten Temperatur von zwischen 80°C und 150°C durchgeführt wird.

16. Das Verfahren nach irgendeinem der Ansprüche 13-15, wobei der Blasformschritt (Figuren 2c, 3) bei erhöhten Temperaturen von zwischen 80°C und 150°C durchgeführt wird.

17. Das Verfahren nach irgendeinem der Ansprüche 13-16, wobei der Zuführschritt (Fig. 2a) ein Vorformlingsrohr aus einem Nylon oder einem Polyamid zuführt, und worin der Schritt des axialen Ausdehnens (Fig. 2b) des zugeführten Vorformlings den Vorformling auf wenigstens das Zweifache der Länge des Vorformlings vor dem axialen Ausdehnungsschritt dehnt.

18. Das Verfahren nach irgendeinem der Ansprüche 13-17, weiter einschließend das Dazwischenanordnen eines Wärmeisolationsmittels (52, 53) zwischen die Freiblaskammer (55) und die Blasformkammer (56), so daß die erhöhte Temperatur des radialen Expansionsschrittes im wesentlichen begrenzt ist auf den Abschnitt des Vorformlings, der radial expandiert werden soll.

19. Das Verfahren nach einem der Ansprüche 13-18, wobei der Schritt des axialen Ausdehnens innerhalb der Blasformkammer (56) axiales Ausdehnen (Fig. 2d) des geformten Vorformlings einschließt, bis sein Durchmesser vermindert ist und Blasformen (Fig. 2e) des axial ausgedehnten geformten Vorformlings.

20. Das Verfahren nach irgendeinem der Ansprüche 13-19, weiter einschließend Steuern des Ausmaßes der radialen Expansion, welche durch den Blasformschritt (Figuren 2c, 3) erreicht wird durch Ausführen des Blasformens bei einer erhöhten Temperatur und dann Reduzieren der Temperatur innerhalb der Freiblaskammer (55) auf Raumtemperatur oder darunter, sobald ein gewünschtes Ausmaß an radialer Expansion erreicht ist.

21. Das Verfahren nach irgendeinem der Ansprüche 19-20, ferner einschließend Erhöhen der Temperatur des radial expandierten und axial ausgedehnten Vorformlings innerhalb der Blasformkammer (56) und Infundieren des unter Druck stehenden Fluids in einen Abschnitt des gedehnten Vorformlings, welcher stromaufwärts von der Freiblaskammer (55) liegt, wodurch das unter Druck stehende Fluid durch den Vorformling innerhalb der Freiblaskammer (55) und dann in den radial expandierten und axial ausgedehnten Vorformling innerhalb der Blasformkammer (56) fließt; und Abschließen des Vorformlings zwischen der Freiblaskammer (55) und der Blasformkammer (56), um eine Passage von unter Druck stehendem Fluid dazwischen zu vermeiden.

## Revendications

1. Appareil de moulage pour la fabrication de ballons pour dispositifs médicaux, l'appareil incluant une chambre de soufflage (56) présentant un orifice d'entrée et un orifice de sortie, des moyens d'introduction d'une ébauche (41) par l'orifice d'entrée et l'orifice de sortie de ladite chambre de soufflage (56), des moyens (46, 67, 47, 68) pour allonger axialement ladite ébauche dans une première direction alors qu'une première partie de ladite ébauche se trouve à l'intérieur de la chambre de soufflage (56), des moyens (43, 44, 45) pour amener un fluide sous pression dans ladite ébauche (41) et pour effectuer la dilatation radial de ladite première partie de l'ébauche (41) à l'intérieur de ladite chambre de soufflage (56) grâce au passage d'un fluide préssurisé des moyens d'alimentation (43, 44, 45) dans ladite première partie de l'ébauche, caractérisé par:
une chambre libre (55) présentant un orifice d'entrée et un orifice de sortie en amont de ladite chambre de soufflage (56),
lesdits moyens d'alimentation de l'ébauche (41) alimentant également celle-ci à travers l'orifice d'entrée et l'orifice de sortie de ladite chambre libre (55),
lesdits moyens (46, 67, 47, 68) pour allonger axialement ladite ébauche dans une première direction étirant également ladite ébauche alors qu'une seconde portion de celle-ci se trouve à l'intérieur de la chambre libre (55),
lesdits moyens (43, 44, 45) d'alimentation du fluide préssurisé alimentant en même temps en amont ladite chambre libre (55) par l'orifice d'entrée,
ladite chambre libre (55) présentant des moyens pour permettre l'élongation d'une portion définie de ladite ébauche dans une seconde direction en dilatant radialement ladite seconde portion de l'ébauche (41) se trouvant à l'intérieur lors du passage à l'intérieur d'un fluide sous pression venant desdits moyens d'alimentation (43, 44, 45), et
par des moyens pour isoler thermiquement (51, 52, 53, 54) ladite chambre libre (55) des autres composants de l'appareil y compris ladite chambre de soufflage (56).

2. Appareil de moulage selon la revendication 1, incluant de plus des moyens de transfert de chaleur (62) pour élever la température de la seconde portion de l'ébauche (41) à l'intérieur de ladite chambre de soufflage (55) durant ladite d'opération de dilatation radiale de cette seconde portion de l'ébauche (41) dans le but de former une portion élargie radialement de l'ébauche (41).

3. Appareil de moulage selon la revendication 1 ou 2, dans lequel les moyens de transfert de chaleur (62, 63, 64, 65) abaissent la température de ladite portion de l'ébauche élargie radialement de façon telle que les moyens d'élongation axiale (46, 67, 47, 68) orientent axialement l'ébauche (41) lorsque celle-ci est à la température ambiante ou à une température inférieure.

4. Appareil de moulage selon l'une des revendications 1 à 3, dans lequel ladite ébauche (41) est en nylon ou en polyamide et dans lequel lesdits moyens d'élongation axiale (46, 67, 47, 68) allongent axialement ladite ébauche présentant une température ambiante ou une température inférieure jusqu'à au moins deux fois la longueur de l'ébauche (41), avant de procéder à ladite élongation axiale.

5. Appareil de moulage selon l'une quelconque des revendications 1 à 4, incluant de plus des moyens de transfert de chaleur (65) pour élever la température de la première portion de l'ébauche (41) à l'intérieur de ladite chambre de soufflage (56) au cours de ladite dilatation radiale de la première portion de l'ébauche (41) et permettant de refroidir ensuite ladite portion de l'ébauche (41).

6. Appareil de moulage selon la revendication 5, dans lequel lesdits moyens de transfert de chaleur confèrent une température comprise entre 0°C et 150°C à ladite chambre de soufflage (56).

7. Appareil de moulage selon l'une quelconque des revendications 1 à 6, dans lequel lesdits moyens d'isolation thermique (51, 52, 53, 54) desdites chambres (55, 56) incluent des chambres de condensation essentiellement juxtaposées aux orifices d'entrée et de sortie de ladite chambre libre (55) et de ladite chambre de soufflage (56).

8. Appareil de moulage selon l'une quelconque des revendications 1 à 7 dans lequel lesdits moyens d'élongation axiale (46, 67, 47, 68) incluent des moyens de serrage amont (46, 67) pour prévenir tout mouvement longitudinal de l'ébauche (41) et des moyens de serrage aval (47, 68) pour translater longitudinalement l'ébauche et pour empêcher essentiellement le passage de fluide sous pression, lesdits moyens de serrage amont (46, 67) étant prévus en amont de ladite chambre libre (55), et lesdits moyens de serrage aval (47, 68) étant prévus en aval de ladite chambre de soufflage (56).

9. Appareil de moulage selon la revendication 8, incluant de plus des moyens de serrage intermédiaire (48) pour empêcher essentiellement le passage de fluide sous pression à l'intérieur de l'ébauche (41) entre ladite chambre libre (55) et ladite chambre de soufflage (56).

10. Appareil de moulage selon l'une quelconque des revendications 1 à 9, incluant de plus des moyens (69) pour limiter la dilatation radiale de l'ébauche provoquée par ladite chambre libre (55).

11. Appareil de moulage selon la revendication 10, dans lequel lesdits moyens de limitation de la dilatation incluent un élément (69) mis en oeuvre pour coopérer en une zone prédéterminée avec ladite ébauche (41) lorsque celle-ci se dilate à l'intérieur de ladite chambre libre (55).

12. Appareil de moulage selon la revendication 11, dans lequel ledit élément (69) des moyens de contrôle active les moyens de transfert de chaleur pour abaisser la température de l'ébauche à l'intérieur de la chambre libre (55) à partir d'une température de soufflage de 75°C jusqu'à une température plus basse allant jusqu' à l'ambiance.

13. Procédé de fabrication de ballons pour dispositifs médicaux, le procédé incluant les étapes consistant à:
amener (Fig. 2a) une ébauche allongée (41) dans un appareil de moulage incluant une chambre de soufflage (56) dans laquelle l'étirement mécanique de ladite ébauche dans deux directions est effectuée;
étendre axialement (Fig. 2b) l'ébauche pour l'allonger longitudinalement à l'intérieur de ladite chambre de soufflage (56) pour obtenir une longueur d'ébauche allongée axialement;
dilater radialement (Fig. 2c) ladite ébauche allongée axialement à l'intérieur de ladite chambre de moulage (56) en insufflant un fluide sous pression à l'intérieur de celle-ci pour provoquer une expansion radiale de la longueur d'ébauche;
caractérisé en ce que
ladite étape d'amenée (Fig 2a) inclue le passage de l'ébauche (41) à travers une chambre libre (55) en amont de la chambre de soufflage (56); et en ce qu'il consiste à
étirer axialement (Fig 2b) ladite ébauche (41) à l'intérieur de ladite chambre libre (55) pour allonger longitudinalement cette ébauche (41) à l'intérieur de ladite chambre libre (55);
souffler (Fig. 2c, 3) ladite longueur d'ébauche étendue axialement en insufflant un fluide sous pression à l'intérieur de celle-ci alors qu'elle se trouve à l'intérieur de la chambre libre (55) pour provoquer en même temps que l'alimentation, par étirement axial et dilatation radiale, une ébauche moulée dans la forme d'un ballon pour dispositif médical.

14. Procédé selon la revendication 13, dans laquelle ladite étape d'étirement axial (Fig. 2b) à l'intérieur de ladite chambre libre (55) et de ladite chambre de soufflage (56) sont effectuées à la température ambiante.

15. Procédé selon les revendications 13 ou 14, dans lequel ladite étape de dilatation radiale (Fig. 2 c) est effectuée à une température élevée comprise entre 80°C et 150°C.

16. Procédé selon l'une quelconque des revendications 13 à 15, dans lequel ladite étape de soufflage (Fig. 2c, 3) est effectuée à une température élevée comprise entre 80°C et 150°C.

17. Procédé selon l'une quelconque des revendications 13 à 16, dans lequel ladite étape d'alimentation (Fig. 2a) consiste à amener un tube d'ébauche en nylon ou en polyamide, et dans lequel ladite étape consistant à étirer axialement (Fig 2b) l'ébauche, permet d'allonger l'ébauche pour lui conférer une longueur au moins égale à deux fois la longueur de l'ébauche avant l'étape d'étirement axial.

18. Procédé selon l'une quelconque des revendications 13 à 17, incluant de plus l'étape consistant à interposer des moyens d'isolation thermique (52, 53) entre la chambre libre (55) et la chambre de soufflage (56) de façon telle que la température élevée de l'étape de dilatation radiale soit essentiellement confinée à la partie de l'ébauche devant être dilatée radialement.

19. Procédé selon les revendications 13 à 18, dans lequel ladite étape d'extension axiale à l'intérieur de ladite chambre de soufflage (56) consiste à étendre axialement (Fig 2d) ladite ébauche moulée jusqu'à ce que son diamètre diminue et à souffler (Fig 2e) ladite ébauche moulée allongée axialement.

20. Procédé selon l'une quelconque des revendications 13 à 19, incluant de plus l'étape consistant à contrôler l'importance de la dilatation radiale effectuée lors de ladite étape de soufflage (Fig. 2c, 3) en effectuant le soufflage à une température élevée puis en réduisant la température à l'intérieur de la chambre libre (55) jusqu'à la température ambiante ou jusqu'à une température plus faible dès qu'une dilatation radiale suffisante est obtenue.

21. Procédé selon l'une quelconque des revendications 19 ou 20, incluant de plus une étape consistant à augmenter la température de l'ébauche étirée axialement et dilatée radialement à l'intérieur de la chambre de soufflage (56) et à insuffler le fluide sous pression à l'intérieur d'une portion de l'ébauche allongée se situant en aval de la chambre libre (55) dans laquelle le fluide sous pression s'écoule à travers l'ébauche à l'intérieur de la chambre libre (55) puis à l'intérieur de l'ébauche étirée axialement et dilatée radialement à l'intérieur de la chambre de moulage (56); et consistant ensuite à fermer l'ébauche entre la chambre libre (55) et la chambre de soufflage (56) pour empêcher le passage du fluide sous pression à l'intérieur de celle-ci.
